(19) Europäisches Patentamt
European Patent Office
Office européen des brevets

(11) Veröffentlichungsnummer: **0 185 882**
**B1**

(12) # EUROPÄISCHE PATENTSCHRIFT

(45) Veröffentlichungstag der Patentschrift: **13.09.89**

(51) Int. Cl.⁴: **C 07 F 9/65**

(21) Anmeldenummer: **85113559.0**

(22) Anmeldetag: **25.10.85**

(54) **Verfahren zur Herstellung von optisch aktivem 1-Benzyl-3,4-bis-(diphenylphosphino)-pyrrolidin.**

(30) Priorität: **19.12.84 DE 3446303**

(43) Veröffentlichungstag der Anmeldung: **02.07.86 Patentblatt 86/27**

(45) Bekanntmachung des Hinweises auf die Patenterteilung: **13.09.89 Patentblatt 89/37**

(84) Benannte Vertragsstaaten: **AT BE CH DE FR GB IT LI NL SE**

(56) Entgegenhaltungen:
**EP-A-0 151 282**

**ANGEWANDTE CHEMIE. "INTERNATIONAL EDITION", Band 23, Nr. 6, Juni 1984, Seiten 435-436, Verlag Chemie GmbH, Weinheim, DE; U. NAGEL: "Asymmetric hydrogenation of alpha-(acetylamino)cinnamic acid with a novel rhodium complex; the design of an optimal ligand"**

(73) Patentinhaber: **Degussa Aktiengesellschaft, Weissfrauenstrasse 9, D-6000 Frankfurt am Main 1 (DE)**

(72) Erfinder: **Andrade, Juan, Dr. Dipl.- Chem., Hungerweg 5, D-8752 Kleinostheim (DE)**
Erfinder: **Prescher, Günter, Dr. Dipl.- Chem., Liesingstrasse 2, D-6450 Hanau 9 (DE)**
Erfinder: **Nagel, Ulrich, Dr. Dipl.- Chem., Freiherrnstrasse 12, D-8061 Weichs (DE)**

**EP 0 185 882 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung von optisch aktivem 1-Benzyl-3,4-bis-(diphenylphosphino)-pyrrolidin der Formel

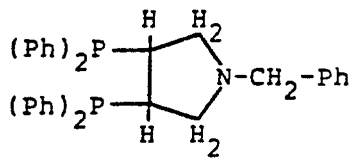

(I),

in der Ph jeweils einen Phenylrest bedeutet.

Dieses Pyrrolidin-Derivat, es als chiralen Liganden enthaltende Rhodiumkomplexe und deren Verwendung als Katalysatoren für die homogene asymmetrische Hydrierung unsubstituierter oder β-substituierter α-Acylamino-acrylsäuren sind in der älteren, aber nicht vorveröffentlichten EP-A-0 151 282 beschrieben, die unter Artikel 54 (3) EPÜ fällt. Das dort ebenfalls beschriebene Herstellungsverfahren für das Pyrrolidin-Derivat ist außerordentlich vielstufig und infolgedessen recht umständlich und langwierig.

Aus "ANGEWANDTE CHEMIE, INTERNATIONAL EDITION, Band 23, Nr. 6, Juni 1984, Seiten 435 bis 436, Verlag Chemie GmbH, Weinheim" ist ferner die Herstellung des bei dem in EP-A-0 151 282 beschriebenen Verfahren als Zwischenprodukt dienenden 1-Benzoyl-3,4-bis-(diphenylphosphino)-pyrrolidins bekannt.

Es wurde nun ein neues Herstellungsverfahren gefunden, durch das optisch aktives 1-Benzyl-3,4-bis-(diphenylphosphino)pyrrolidin in nur noch drei Verfahrensstufen auf relativ einfache Weise hergestellt werden kann.

Das erfindungsgemäße Verfahren ist dadurch gekennzeichnet, daß man

a) optisch aktives 1-Benzyl-2,5-dioxo-3,4-dihydroxypyrrolidin der Formel

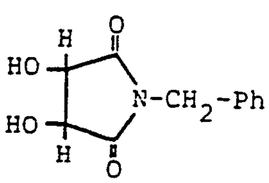

(II)

bei einer Temperatur zwischen -50 und +170 °C in einem inerten Lösungsmittel und in Gegenwart der 0,1- bis 20-fachen molaren Menge an Bortrifluorid mit der 1- bis 20-fachen molaren Menge an Natriumboranat zu 1-Benzyl-3,4-dihydroxy-pyrrolidin der Formel

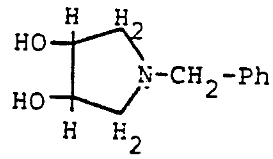

(III)

reduziert,

b) dieses bei einer Temperatur zwischen -40 und +100°C in Gegenwart der 2- bis 20-fachen molaren Menge an einem tertiären Amin mit 2 bis 10 Äquivalenten Methansulfonsäureanhydrid oder -chlorid in das 1-Benzyl-3,4-dimethansulfonyl-pyrrolidin der Formel

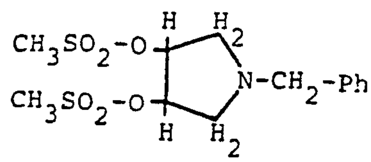

(IV)

umwandelt und

c) dieses bei einer Temperatur zwischen -20 und +40 °C in einem inerten Lösungsmittel und unter Sauerstoffausschluß mit der 2- bis 6-fachen molaren Menge eines Alkalimetalldiphenylphosphids umsetzt.

Das in der Verfahrensstufe a) als Ausgangsmaterial dienende optisch aktive 1-Benzyl-2,5-dioxo-3,4-dihydroxy-pyrrolidin der Formel (II) kann einfach in an sich bekannter Weise dadurch hergestellt werden, daß man optisch aktive Weinsäure mit Benzylamin kondensiert. Setzt man dabei L-Weinsäure ein, ist das Endprodukt des erfindungsgemäßen Verfahrens das 1-Benzyl-3,4-(R,R)-bis-(diphenylphosphino)-pyrrolidin, geht man von D-Weinsäure aus, entsteht in entsprechender Weise das 1-Benzyl-3,4-(S,S)-bis-(diphenylphosphino)-pyrrolidin.

Die Verfahrensstufe a) wird vorzugsweise bei einer Temperatur zwischen 0 und 100°C durchgeführt. Geeignete inerte Lösungsmittel sind solche, in denen Bortrifluorid ($BF_3$) und Natriumboranat ($NaBH_4$) löslich sind, wie Diethylether, Diisopropylether, 1,4-Dimethoxybutan, Tetrahydrofuran oder Diglykoldimethylether. Das Bortrifluorid kann als Gas oder als Etherat-Komplex eingesetzt werden. Es wird vorzugsweise in der 1- bis 6-fachen molaren Menge angewandt. Das Natriumboranat wird zweckmäßigerweise unter Eiskühlung in kleinen Portionen langsam zugegeben. Es wird vorzugsweise in der 1- bis 4-fachen molaren Menge angewandt.

Die Aufarbeitung des rohen Reaktionsgemisches der Verfahrensstufe a) kann beispielsweise so vorgenommen werden, daß man überschüssige Salzsäure langsam zutropft bis die

Gasentwicklung beendet ist und dann noch einmal für kurze Zeit auf höhere Temperatur, z. B. 70°C, erwärmt. Besonders vorteilhaft ist es, wenn man dann einen Komplexbildner für die im Reaktionsgemisch enthaltenen Borverbindungen zusetzt und noch einmal einige Zeit auf höhere Temperatur, z. B. 100°C, erhitzt. Solche Komplexbildner sind beispielsweise Polyhydroxylverbindungen mit benachbarten Hydroxylgruppen, wie Glycerin, Zuckeralkohole oder Zucker, insbesondere Monosaccharide, und/oder Alkalimetallfluoride, insbesondere Natriumfluorid. Diese Stoffe werden zweckmäßigerweise in einem molaren Überschuß über die insgesamt eingesetzten Borverbindungen angewandt. Nach dem Abkühlen wird dann mit Natronlauge neutralisiert. Die wäßrige Phase wird abgetrennt und verworfen, die organische Phase unter vermindertem Druck eingedampft. Der Rückstand wird in Wasser aufgenommen und mit einem geeigneten Lösungsmittel, vorzugsweise mit Essigsäureethylester, erschöpfend extrahiert. Die vereinigten Extrakte werden unter vermindertem Druck auf ein möglichst kleines Volumen eingeengt und auf eine niedrige Temperatur, z. B. 5°C, abgekühlt. Beim Stehenlassen kristallisiert dann das 1-Benzyl-3,4-dihydroxy-pyrrolidin der Formel (III) aus.

Die Verfahrensstufe b) wird vorzugsweise bei einer Temperatur zwischen -20 und +20°C durchgeführt. Das tertiäre Amin wird zweckmäßigerweise in der gleichen molaren Menge eingesetzt wie das Mesylierungsmittel. Geeignete tertiäre Amine sind beispielsweise Triethylamin, Tris-n-butylamin oder Pyridin. Als Mesylierungsmittel wird das Methansulfonsäurechlorid bevorzugt angewandt. Gegebenenfalls kann die Umsetzung auch in einem inerten Lösungsmittel vorgenommen werden. Geeignete Lösungsmittel sind insbesondere aliphatische Kohlenwasserstoffe, wie n-Pentan oder n-Hexan; Methylenchlorid oder Tetrachlorkohlenstoff. Das Mesylierungsmittel wird zweckmäßigerweise zu dem vorgelegten 1-Benzyl-3,4-dihydroxy-pyrrolidin der Formel (III) und dem tertiären Amin, gegebenenfalls als Lösung in einem inerten Lösungsmittel, unter Kühlung langsam zugetropft. Das Mesylierungsmittel wird vorzugsweise ebenso wie das tertiäre Amin in der 2- bis 4-fachen molaren Menge angewandt. Nach beendeter Zugabe des Mesylierungsmittels empfiehlt es sich, das Reaktionsgemisch noch etwa 30 Minuten lang bei Raumtemperatur oder erhöhter Temperatur zu rühren.

Die Aufarbeitung des rohen Reaktionsgemisches der Verfahrensstufe b) kann beispielsweise so vorgenommen werden, daß man es zunächst mit Wasser auswäscht und nach dem Abtrennen des Waschwassers unter kräftigem Rühren mit überschüssiger verdünnter Salzsäure versetzt. Dann wird die Methylenchloridphase abdekantiert und verworfen. Die salzsaure Wasserphase wird bei etwa Raumtemperatur mit Natronlauge neutralisiert und der sich dabei abscheidende ölige Niederschlag wird mit Methylenchlorid extrahiert. Das Lösungsmittel wird unter vermindertem Druck eingedampft und der Rückstand wird aus Ethanol umkristallisiert.

Die Verfahrensstufe c) wird vorzugsweise bei einer Temperatur zwischen -10 und +10°C durchgeführt. Geeignete inerte Lösungsmittel sind beispielsweise Ether, wie Diethylether, Di-n-butylether, Tetrahydrofuran oder Dioxan. Der Luftsauerstoff wird durch Anwendung einer Inertgasatmosphäre von beispielsweise Stickstoff oder Argon streng ausgeschlossen. Als Alkalimetalldiphenylphosphide werden bevorzugt die des Lithiums, Natriums oder Kaliums angewandt. Sie werden vorzugsweise in der 2- bis 3-fachen molaren Menge eingesetzt. Zweckmäßigerweise werden sie frisch in situ aus dem Alkalimetall und aus Chlordiphenylphosphin hergestellt.

Das 1-Benzyl-3,4-dimethansulfonyl-pyrrolidin wird zu der vorgelegten Lösung des Alkalimetalldiphenylphosphids zugegeben.

Die Aufarbeitung des rohen Reaktionsgemisches der Verfahrensstufe c) kann beispielsweise so vorgenommen werden, daß man es mit einer geringen Menge Wasser versetzt und es dann unter vermindertem Druck eindampft. Der Rückstand wird in Diethylether aufgenommen und die Etherlösung wird mit Wasser ausgewaschen, das dann verworfen wird. Anschließend wird das in der Lösung enthaltene 1-Benzyl-3,4-bis-(diphenylphosphino)-pyrrolidin der Formel (I) als Hydrochlorid isoliert. Dazu wird die Etherlösung mit verdünnter Salzsäure versetzt, wodurch ein wachsartiger Niederschlag entsteht. Die flüssige Phase wird abdekantiert und der Rückstand wird aus Isopropylalkohol umkristallisiert.

Das Hydrochlorid ist in trockenem Zustand gut haltbar. Im Bedarfsfalle kann daraus durch Behandeln mit einem Alkalimetallhydroxid, -hydrogencarbonat oder -carbonat oder mit einem tertiären Amin die freie Base gewonnen werden.

Die Erfindung wird durch das nachfolgende Beispiel für den Fall der Herstellung von 1-Benzyl-3,4-(R,R)-bis-(diphenylphosphino)-pyrrolidin-Hydrochlorid aus 1-Benzyl-2,5-dioxo-3,4-(R,R)-dihydroxy-pyrrolidin näher erläutert. Alle Prozentangaben bedeuten, sofern nicht anders angegeben, Gewichtsprozente.

**Beispiel:**

Verfahrensstufe a)

200 g (2,94 Mol) gasförmiges Bortrifluorid wurden bei 0°C in 1500 ml Diglykoldimethylether eingeleitet. Die Lösung wurde mit 165 g (0,76 Mol) 1-Benzyl-2,5-dioxo-3,4-(R,R)-dihydroxy-pyrrolidin versetzt und es wurden unter Eiskühlung 75 g (2,0 Mol) $NaBH_4$ in kleinen Portionen langsam zugegeben, wodurch eine exotherme Reaktion unter Gasentwicklung einsetzte. Nach beendeter Zugabe wurde das Reaktionsgemisch noch zwei Stunden unter Rühren auf 70°C gehalten und dann auf Raumtemperatur abgekühlt.

Anschließend wurden 900 ml 20 %-ige Salzsäure unter Gasentwicklung langsam zugetropft und das Reaktionsgemisch wurde noch einmal 15 Minuten unter Rühren auf 70°C erwärmt. Dann wurden unter Rühren 400 g Natriumfluorid auf einmal zugegeben und das Reaktionsgemisch wurde weitere 30 Minuten auf 100°C erhitzt. Nach dem Abkühlen auf 20°C wurde das Reaktionsgemisch mit 950 ml 20 %-iger Natronlauge neutralisiert. Die wäßrige Phase wurde abgetrennt und verworfen, die organische Phase unter vermindertem Druck eingedampft. Der Rückstand wurde in 600 ml Wasser aufgenommen und die Lösung wurde dreimal mit je 600 ml Essigsäureethylester extrahiert. Die vereinigten Extrakte wurden unter vermindertem Druck auf ein Volumen von 500 ml eingeengt, auf 5°C gekühlt und bei dieser Temperatur über Nacht stehen gelassen. Es bildeten sich 125,4 g (87 % der Theorie) an nahezu farblosen Kristallen von 1-Benzyl-3,4-(S,S)-dihydroxy-pyrrolidin mit einem Schmelzpunkt von 100°C.

$[\alpha]^{20}_D$: +32,3° (c = 1,5; Methanol)

Verfahrensstufe b)

77 g (0 4 Mol) 1-Benzyl-3, 4-(S,S)-dihydroxy-pyrrolidin und 90 g (0,88 Mol) Triethylamin wurden in 500 ml Methylenchlorid gelöst. Zu der Lösung wurden bei 5 bis 10°C 100,8 g (0,88 Mol) Methansulfonylchlorid langsam zugetropft, das Reaktionsgemisch wurde auf Raumtemperatur kommen gelassen und noch 30 Minuten gerührt.

Dann wurde das Reaktionsgemisch zweimal mit je 150 ml Wasser gewaschen und das Waschwasser verworfen. Die Methylenchloridlösung wurde mit 2 l 1N HCl kräftig gerührt, anschließend wurde das Methylenchlorid abdekantiert und verworfen. Die Wasserphase wurde bei Raumtemperatur mit 20 %-iger Natronlauge neutralisiert. Der gebildete ölige Niederschlag wurde zweimal mit je 300 ml Dichlormethan extrahiert. Aus den vereinigten Extrakten wurde unter vermindertem Druck das Methylenchlorid abgedämpft und der Rückstand wurde aus 200 ml Ethanol umkristallisiert. Es ergaben sich 119 g (85 % der Theorie) an 1-Benzyl-3,4-(S,S)-dimethansulfonyl-pyrrolidin mit einem Schmelzpunkt von 56,5°C.

$[\alpha]^{20}_D$: +36,4° (c = 1; Methanol)

Verfahrensstufe c)

Bis zur späteren. Wasserzugabe wurde die gesamte Umsetzung unter Argon vorgenommen.

25 g Natrium wurden bei 20°C in kleinen Stückchen in 320 ml absolutem Dioxan suspendiert und die Suspension wurde auf Rückflußtemperatur erhitzt. Innerhalb von etwa 45 Minuten wurden 66 g Chlordiphenylphosphin langsam zugetropft und das Reaktionsgemisch wurde noch 2 Stunden auf Rückflußtemperatur gehalten. Dann wurde auf 80°C abgekühlt und mit 450 ml absolutem Tetrahydrofuran verdünnt. Um den Natriumüberschuß zu entfernen, wurde das Reaktionsgemisch über eine mit Glaswolle gefüllte Säule filtriert und das Filtrat wurde auf 0°C abgekühlt.

Dann wurden unter Rühren 41,9 g 1-Benzyl-3,4-(S,S)dimethansulfonyl-pyrrolidin auf einmal zugegeben. Nach einer Stunde wurde das nunmehr gelartige und nur noch schwer rührbare Reaktionsgemisch mit weiteren 300 ml absolutem Tetrahydrofuran verdünnt und über Nacht stehen gelassen, um auf Raumtemperatur zu kommen.

Nach Zugabe von 100 ml Wasser wurde dann bei einer Badtemperatur von 25°C unter vermindertem Druck eingedampft. Der Rückstand wurde in 350 ml Diethylether aufgenommen, zweimal mit je 100 ml Wasser gewaschen, welches dann verworfen wurde, und mit 60 ml 2N HCl versetzt, wodurch sich ein wachsartiger Niederschlag bildete. Die flüssige Phase wurde abdekantiert und der Rückstand wurde aus 170 ml Isopropylalkohol umkristallisiert. Es ergaben sich 30,8 g (45,3 % der Theorie) an 1-Benzyl-3,4-(R,R)-bis-(diphenylphosphino)pyrrolidin-Hydrochlorid mit einem Schmelzpunkt von 102 bis 108°C.

$[\alpha]^{20}_D$: +86,9° (c = 0,88; Methanol)

**Patentansprüche**

1. Verfahren zur Herstellung von optisch aktivem 1-Benzyl-3,4-bis-(diphenylphosphino)-pyrrolidin der Formel

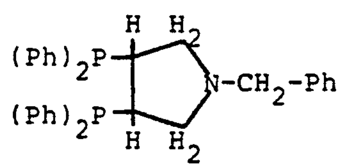 (I),

in der Ph jeweils einen Phenylrest bedeutet, dadurch gekennzeichnet, daß man

a) optisch aktives 1-Benzyl-2,5-dioxo-3,4-dihydroxypyrrolidin der Formel

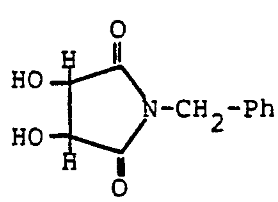 (II),

bei einer Temperatur zwischen -50 und +170°C in einem inerten Lösungsmittel und in Gegenwart der 0,1- bis 20-fachen molaren Menge an Bortrifluorid mit der 1- bis 20-fachen molaren Menge an Natriumboranat zu 1-Benzyl-3,4-dihydroxy-pyrrolidin der Formel

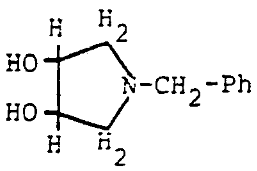

(III)

reduziert,

b) dieses bei einer Temperatur zwischen -40 und +100°C in Gegenwart der 2- bis 20-fachen molaren Menge an einem tertiären Amin mit 2 bis 10 Äquivalenten Methansulfonsäureanhydrid oder -chlorid in das 1-Benzyl-3,4-dimethansulfonyl-pyrrolidin der Formel

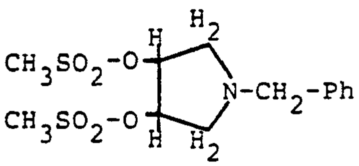

(IV)

umwandelt und

c) dieses bei einer Temperatur zwischen -20 und +40°C in einem inerten Lösungsmittel und unter Sauerstoffausschluß mit der 2- bis 6-fachen molaren Menge eines Alkalimetalldiphenylphosphids umsetzt.

2. Verfahren nach Anspruch 1, dadurch gekennzeichnet, daß man die Verfahrensstufe a) bei einer Temperatur zwischen 0 und 100°C durchführt.

3. Verfahren nach Anspruch 1 oder 2, dadurch gekennzeichnet, daß man in der Verfahrensstufe a) das Bortrifluorid in der 1-bis 6-fachen molaren Menge einsetzt.

4. Verfahren nach einem der Ansprüche 1 bis 3, dadurch gekennzeichnet, daß man in der Verfahrensstufe a) das Natriumboranat in der 1- bis 4-fachen molaren Menge einsetzt.

5. Verfahren nach einem der Ansprüche 1 bis 4, dadurch gekennzeichnet, daß man die Verfahrensstufe b) bei einer Temperatur zwischen -20 und +20 °C durchführt.

6. Verfahren nach einem der Ansprüche 1 bis 5, dadurch gekennzeichnet, daß man in der Verfahrensstufe b) das tertiäre Amin in der 2- bis 4-fachen molaren Menge einsetzt.

7. Verfahren nach einem der Ansprüche 1 bis 6, dadurch gekennzeichnet, daß man in der Verfahrensstufe b) das Methansulfonsäureanhydrid oder -chlorid in einer Menge von 2 bis 4 Äquivalenten einsetzt.

8. Verfahren nach einem der Ansprüche 1 bis 7, dadurch gekennzeichnet, daß man die Reaktionsstufe c) bei einer Temperatur zwischen -10 und +10°C durchführt.

9. Verfahren nach einem der Ansprüche 1 bis 8, dadurch gekennzeichnet, daß man in der Verfahrensstufe c) das Alkalimetalldiphenylphosphid in der 2- bis 3-fachen molaren Menge einsetzt.

## Claims

1. Process for the preparation of optically active 1-benzyl-3,4-bis(diphenylphosphino)-pyrrolidine of the formula

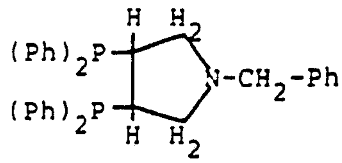

(I)

in which Ph in each case denotes a phenyl radical, characterized in that

a) optically active 1-benzyl-2,5-dioxo-3,4-dihydroxypyrrolidine of the formula

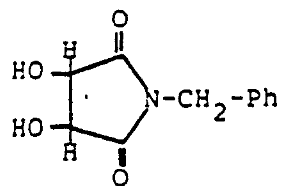

(II)

is reduced at a temperature between -50 and +170°C in an inert solvent and in the presence of a 0.1- to 20-fold molar amount of boron trifluoride using a 1- to 20-fold molar amount of sodium borohydride to give 1-benzyl-3,4-dihydroxypyrrolidine of the formula

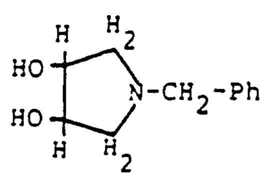

(III)

b) the latter is converted at a temperature of between -40 and +100°C in the presence of a 2- to 20-fold molar amount of a tertiary amine and using 2 to 10 equivalents of methanesulphonic anhydride or chloride into 1-benzyl-3,4-dimethanesulphonylpyrrolidine of the formula

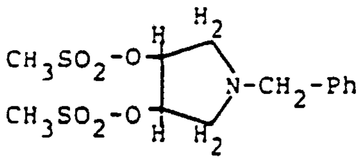

(IV)

and

c) this is reacted at a temperature between -20 and +40°C in an inert solvent and with exclusion of oxygen using a 2- to 6-fold molar amount of an alkali metal diphenylphosphide.

2. Process according to Claim 1, characterized in that process step a) is carried out at a temperature between 0 and 100°C.

3. Process according to Claim 1 or 2, characterized in that in process step a) the boron trifluoride is employed in a 1- to 6-fold molar amount.

4. Process according to one of Claims 1 to 3, characterized in that in process step a) the sodium borohydride is employed in a 1- to 4-fold molar amount.

5. Process according to one of Claims 1 to 4, characterized in that process step b) is carried out at a temperature between -20 and +20°C.

6. Process according to one of Claims 1 to 5, characterized in that in process step b) the tertiary amine is employed in a 2- to 4-fold molar amount.

7. Process according to one of Claims 1 to 6, characterized in that in process step b) the methanesulphonic anhydride or chloride is employed in an amount of 2 to 4 equivalents.

8. Process according to one of Claims 1 to 7, characterized in that reaction step c) is carried out at a temperature between -10 and +10°C.

9. Process according to one of Claims 1 to 8, characterized in that in process step c) the alkali metal diphenylphosphide is employed in a 2- to 3-fold molar amount.

**Revendications**

1. Procédé d'obtention de la 1-benzyl-3,4-bis-(diphénylphosphinol-pyrrolidine optiquement active de formule:

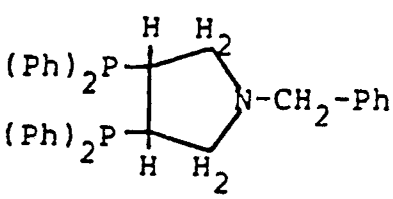

(I)

dans laquelle Ph respectivement représente un reste phényle, caractérisé en ce que

a) l'on reduit la 1-benzyl-2,5-dioxo-3,4-dihydroxy-pyrrolidine optiquement active de formule:

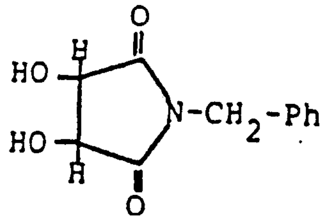

(II)

à une température comprise entre -50° et +170°C dans un solvant inerte et en présence d'une quantité 0,1 à 20 fois molaires de trifluorure de bore, au moyen d'une quantité de 1 à 20 fois molaires de boranate de sodium, en 1-benzyl-3,4-dihydroxy-pyrrolidine de formule:

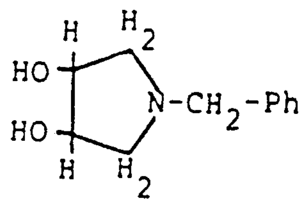

(III)

b) l'on convertit celle-ci à une température comprise entre -40 et +100°C en présence d'une quantité 2 à 20 fois molaires d'amine tertiaire, au moyen de 2 à 10 équivalents d'anhydride ou de chlorure d'acide méthane sulfonique, en 1-benzyl-3,4-diméthanesulfonyloxy-pyrrolidine de formule:

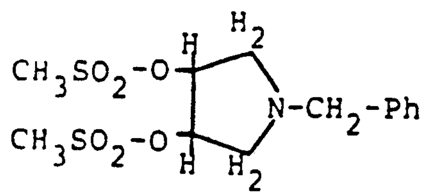

(IV)

c) et on fait réagir celle-ci à une température comprise entre -20 et +40°C dans un solvant inerte et sous exclusion de l'oxygène, avec une quantité 2 à 6 fois molaires d'un diphénylphosphure de métal alcalin.

2. Procédé selon la revendication 1, caractérisé en ce que l'on exécute le stade a) du procédé à une température comprise entre 0 et 100°C,

3. Procédé selon la revendication 1 ou 2, caractérisé en ce que l'on met en jeu dans le stade a) du procédé le trifluorure de bore en quantité 1 à 6 fois molaires.

4. Procédé selon l'une des revendications 1 à 3, caractérisé en ce que l'on met en jeu dans le stade a) du procédé le boranate de sodium en quantité 1 à 4 fois molaires.

5. Procédé selon l'une des revendications 1 à 4,

caractérisé en ce que l'on effectue le stade b) du procédé à une température comprise entre -20 et +20°C.

6. Procédé selon l'une des revendications 1 à 5, caractérisé en ce que l'on met en jeu dans le stade b) du procédé l'amine tertiaire en quantité 2 à 4 fois molaires.

7. Procédé selon l'une des revendications 1 à 6, caractérise en ce que l'on met en jeu dans le stade b) du procédé l'anhydride ou le chlorure de l'acide méthane sulfonique en quantité de 2 à 4 équivalents.

8. Procédé selon l'une des revendications 1 à 7, caractérisé en ce que l'on effectue le stade c) du procédé à une température comprise entre -10 et +10°C.

9. Procédé selon l'une des revendications 1 à 8, caractérisé en ce que l'on met en jeu dans le stade c) du procédé le diphenylphosphure de métal alcalin en quantité 2 à 3 fois molaires.